# EUROPEAN PATENT APPLICATION

(11) **EP 0 547 234 A1**
(43) Date of publication of application: **23.06.1993**
(21) Application number: 92914092.9
(22) Date of filing: 07.07.1992
(51) Int. Cl.: A61K 37/02, A61K 35/12, A61K 35/22

(54) **REMEDY FOR OSTEOPOROSIS**

(30) Priority: 08.07.1991 JP 192714/91
(71) Applicant: THE GREEN CROSS CORPORATION, Osaka-shi Osaka 541 (JP); MORINAGA MILK INDUSTRY CO., LTD., Minato-ku, Tokyo-to 108 (JP)
(72) Inventor: HANAMURA, Takuji, The Green Cross Corporation, Hirakata-shi, Osaka 573 (JP); LEE, Lyang-Ja, The Green Cross Corporation, Hirata--shi, Osaka 573 (JP); MATSUOKA, Yasushi, The Green Cross Corporation, Hirakata-shi, Osaka 573 (JP); SUGIURA, Masanori, The Green Cross Corporation, Hirakata-shi, Osaka 573 (JP)
(74) Representative: Kolb, Helga, Dr. Dipl.-Chem.
(86) International application number: JP9200874
(87) International publication number: WO9300921

(57) **Abstract**

A remedy for osteoporosis containing a monocyte-macrophage colony formation stimulating factor as an active ingredient to be used for promoting normal osteogenesis with reduced side effects to thereby attain radical treatment of osteoporosis by solving the problem presented by the prolonged use of estrogen which is known as a drug for promoting osteogenesis but is dangerous owing to the side effect on the uterus and carcinogenicity.

## Description

### FIELD OF THE INVENTION

This invention relates to a remedy for osteoporosis which contains a monocyte-macrophage colony stimulating factor (M-CSF) as an active ingredient.

### BACKGROUND OF THE INVENTION

Osteoporosis is a disease wherein bone resorption and osteogenesis become imbalanced due to cacochymia, endocrinopathy or aging and, as a result, the amount of bone mass is reduced, thus causing porosis in bones. The symptoms of this disease involve serious dorsolumbar pain and fracture due to the porosis in bones. It is sometimes observed that osteoporosis leads aged persons to becoming terminally bed-ridden or to death.

It is considered that osteoporosis may be treated with drugs capable of relieving dorsolumbar pain, suppressing bone resorption or promoting bone formation. Each of the drugs conventionally employed for treating osteoporosis such as calcitonin, vitamin D and calcium preparations has a mechanism of action of relieving dorsolumbar pain and suppressing bone resorption. Namely, none of them can positively promote bone formation. Bone formation and bone resorption should be essentially well-balanced. Therefore, it is feared that the suppression of bone resorption might suppress bone formation. Estrogen is known as a drug capable of promoting bone formation. However it exerts some side effects on the uterus and exhibits carcinogenesis, which makes the prolonged administration of this compound undesirable. Thus, it has been urgently required to develop a drug for radically treating osteoporosis which can promote normal bone formation while exhibiting reduced side effects.

On the other hand, colony stimulating factors (hereinafter referred to simply as CSF) are endogenous factors which promote the proliferation and differentiation of granulocytes and macrophages. CSFs, which act on granulocyte/macrophage colony forming units (GM-CFU) of bone marrow, may be classified into 1) granulocyte-CSF (G-CSF) forming granulocytes; 2) monocyte-macrophage-CSF (M-CSF) forming monocytes-macrophages; and 3) granulocyte macrophage-CSF (GM-CSF) forming both of granulocytes and macrophages.

It is an object of the present invention to provide a remedy for osteoporosis, for completely treating osteoporosis, which can promote normal bone formation while exhibiting reduced side effects.

Under the above-mentioned circumstances, the present inventors have conducted extensive studies. As a result, they have successfully found that M-CSF is effective in the treatment of osteoporosis, thus completing the present invention.

### DISCLOSURE OF THE INVENTION

The present invention provides a remedy for osteoporosis containing a monocyte-macrophage colony stimulating factor as an active ingredient.

The M-CSF of the present invention is not particularly restricted so long as it is a protein factor capable of promoting the proliferation and differentiation of macrophages.

Examples of the above-mentioned M-CSF include those which can promote the proliferation and differentiation of monocytes-macrophages and are selected from known M-CSFs and CSF-HU (which is also called human urine-derived CSF).

The M-CSF may be prepared by purifying from human urine, incubating M-CSF-producing cells or employing genetic engineering techniques.

Particular examples of the method for preparing the M-CSF include those disclosed in JP-A-54-140707, JP-A-63-54398, JP-A-63-290900, JP-A-63-198700, JP-A-63-250400 and JP-A-1-22899.

Further, peptide fragments having the M-CSF activity and derivatives of said fragments are also usable in the present invention. Such a fragment may be obtained by desaccharification or digestion with the use of known enzymes. Alternatively, peptide fragments having the M-CSF activity constructed by genetic engineering techniques may be employed.

The M-CSF of the present invention may be dissolved in, for example, physiological saline for injection or distilled water for injection to give a concentration of 10³ to 10⁶ U/ml and then administered by, for example, intravenous injection, intravenous drip infusion, intramuscular injection or subcutaneous injection.

It may be administered to an adult in a single dose of from 10⁵ to 2 x 10⁷ U from once to several times per day for 1 to 14 days, though the dose may be appropriately controlled depending on the symptoms.

### BEST MODE TO PRACTICE THE INVENTION

To further illustrate the present invention in greater detail, and not by way of limitation, the following Example is provided.

### (Test method)

Female rats aged 7 to 8 months were subjected to ovariectomy and bisciatic neurotomy to thereby prepare osteoporosis rats. From the next day of the operation, 5 x 10⁵ or 5 x 10⁶ U/kg of an M-CSF was subcutaneously injected into the animals every two days.

This M-CSF was a colony stimulating factor purified from human urine which was commonly called Mirimostim. It was a glycoprotein of a molecular weight of about 84,000 and consisted of protein homo-dimers comprising 214 amino acid residues (C₁₀₅₈H₁₆₄₉N₂₇₇O₃₄₀S₁₄) [cf. "Kiso to Rinsho", 22, No. 8, 66 - 78 (1988)].

On the other hand, a vehicle was exclusively given to an osteoporosis group and a Sham group. After 12 weeks, the femurs of the rats were taken out and fixed with 70 % ethanol for 2 weeks. After the fixation, muscles around the bones were removed to thereby leave the femurs alone and then the bone mineral content and the bone weight were measured. Each group had 5 animals.
- Group 1:: Sham group.
- Group 2:: Osteoporosis group.
- Group 3:: M-CSF/low-dose group (5 x 10⁵ U/kg).
- Group 4:: M-CSF/high-dose group (5 x 10⁶ U/kg).

### (1) Measurement of bone mineral content:

The content of bone mineral was measured and analyzed with a bone mineral content analyzer. The obtained data were analyzed by the DEXA method and thus the total bone mineral content (BMC), the total area (AREA) and the average bone mineral density (BMD) were calculated.

### (2) Measurement of bone weight:

The volume, the dry weight (Dry wt.) and the ash weight (Ash wt.) were measured. The obtained data were given after correcting with the volume.

### (Test Results)

The following tables summarize the test results. The average bone mineral density (BMD) of the group 3 was larger than that of the group 2 but smaller than that of the group 4. The BMD of the group 4 was close to that of the group 1. A similar tendency was observed in the case of the bone weight. Namely, both of the Dry wt. and Ash wt. of the group 3 were larger than those of the group 2 but smaller than those of the group 4. The Dry wt. and Ash wt. of the group 4 were close to those of the group 1. These results have proved that the M-CSF is effective in treating osteoporosis.

| Bone mineral content | | | |
|---|---|---|---|
| | BMC (mg) | AREA (cm²) | BMD (mg/cm²) |
| Group 1 | 320.7±14.9 | 2.106±0.044 | 151.9±4.7 |
| Group 2 | 229.9±12.4 | 2.090±0.086 | 109.8±2.1 |
| Group 3 | 276.0±10.1 | 2.131±0.060 | 129.5±3.1 |
| Group 4 | 315.6± 6.8 | 2.151±0.033 | 146.7±2.3 |

| Bone weight: | | |
|---|---|---|
| | Dry wt./vol (mg/cm³) | Ash wt./vol (mg/cm³) |
| Group 1 | 1198±21 | 793±24 |
| Group 2 | 953±25 | 665±16 |
| Group 3 | 998±15 | 722±14 |
| Group 4 | 1108±21 | 787±13 |

### INDUSTRIAL APPLICABILITY

The present invention provides a remedy for osteoporosis which is clinically useful.

## Claims

1. A remedy for osteoporosis which contains a monocyte-macrophage colony stimulating factor as an active ingredient.

2. A method for treating osteoporosis with the use of a monocyte-macrophage colony stimulating factor.

3. The use of a monocyte-macrophage colony stimulating factor to the production of a drug for treating osteoporosis.
